# EUROPEAN PATENT SPECIFICATION

(11) **EP 1 543 783 B1**
(45) Date of publication and mention of the grant of the patent: **28.11.2007**
(21) Application number: 04257860.9
(22) Date of filing: 16.12.2004
(51) Int. Cl.: A61B 17/12

(54) **Activable bioactive implantable medical device.**
Aktiviebare bioaktive implantierbare medizinische Vorrichtung.
Dispositif médical implantable activable et biologiquement actif.

(30) Priority: 17.12.2003 UA 738477; 22.06.2004 US 874864
(43) Date of publication of application: 22.06.2005
(73) Proprietor: Cordis Neurovascular, Inc., Miami Lakes, Florida 33014 (US)
(72) Inventor: Jones, Donald K, Florida 33351 (US); Lorenzo, Juan A, 106 Terrace, Davie, Florida 33325 (US); Pomeranz, Mark L, Davie, Florida 33325 (US); Sherman, Darren, Florida 33301 (US)
(74) Representative: Tunstall, Christopher Stephen

(56) References cited:
- DE-A1- 19 647 280
- US-A- 5 980 550
- US-A1- 2002 143 348

## Description

### Field of the Invention

The present invention relates to medical implantable devices, and more particularly, to a medical device, such as a vascular occlusive device, which includes a bioactive coating placed on the device for reacting with bodily tissue in order to promote a desired result, such as for example, stimulating tissue growth onto the device.

### Description of the Prior Art

For many years medical devices, such as vascular occlusive devices, have been placed within the vasculature of the human body to occlude, or partially occlude, blood flow through the vasculature. Additionally, such devices have been introduced into aneurysms in order to fill, or partially fill, the aneurysm so as to reduce the pressure which is applied to the interior of the aneurysm in order to prevent further growth or expansion of the aneurysm. These devices may take the form of a coil, such as a helical coil, and are typically placed within the vessel or aneurysm by use of a delivery catheter which is inserted into the vessel and positioned such that the distal end of the delivery catheter is adjacent to a selected site for placement. Once the occlusive device is placed within a blood vessel or aneurysm, surrounding tissue reacts with the "foreign" object and begins to grow into and around the device to provide more complete occlusion of the vessel.

Examples of such delivery catheters are disclosed in U.S. Patent No. 5,108,407, entitled "Method And Apparatus For Placement Of An Embolic Coil" and U.S. Patent No. 5,122,136, entitled "Endovascular Electrolytically Detachable Guidewire Tip For The Electroformation Of Thrombus In Arteries, Veins, Aneurysms, Vascular Malformations And Arteriovenous Fistulas." These patents disclose catheter systems for delivering embolic coils to preselected positions within vessels of the human body in order to treat aneurysms, or alternatively, to occlude a blood vessel at a preselected location.

Occlusive devices which take the form of coils may be helically wound coils, random wound coils, coils wound within coils or other such coil configurations. Examples of various coil configurations are disclosed in U.S. Patent No. 5,334,210, entitled, "Vascular Occlusion Assembly" and U.S. Patent No. 5,382,259, entitled, "Vasoocclusion Coil With Attached Tubular Woven Or Braided Fibrous Covering." Such coils are generally formed from radiopaque metallic materials, such as platinum, gold, tungsten or alloys of these metals. Oftentimes several coils are placed at a given location within a vessel, or within an aneurysm, to more completely occlude, or partially occlude, the flow of blood through the vessel or aneurysm. Thrombus growth onto the coils further enhances the occlusive effect of the coils.

In the past, embolic coils have been placed within the distal end of a delivery catheter and when the distal end of the catheter is properly positioned, the coil may then be pushed out of the end of the catheter with, for example a guidewire, to release the coil at the desired location. This procedure of placement of the embolic coil is conducted under fluoroscopic visualization such that the movement of the coil may be monitored and the coil may be placed at a desired location.

In addition, such coils have been specifically designed to be stretch resistant, such as the vasculature occlusive coil disclosed in U.S. Patent No. 5,853,418, entitled, "Stretch Resistant Vaso-Occlusive Coils (II)" which discloses a helically wound coil having a polymeric stretch resistant member extending through the lumen of the coil and fixedly attached to both ends of the coil to prevent the coil from stretching.

In order to increase the thrombogenicity of an embolic coil, such coils have included a coating, such as collagen, which is applied to the surface of the coil. This concept is disclosed in U.S. Patent No. 5,690,671, entitled, "Embolic Elements And Methods And Apparatus For Their Delivery," which discloses such a collagen coated embolic coil.

In addition, U.S. Patent No. 5,980,550, entitled, "Water-Soluble Coating For Bioactive Vasoocclusive Devices," discloses an embolic coil having a thrombogenic inner coating which serves as a thrombogenic agent and an outer coating of a water-soluble agent which dissolves after placement of the coil in order expose the thrombogenic inner coating to enhance the growth of thrombus into and around the coil. The water-soluble coating prevents the thrombogenic inner coating from coming into contact with the surrounding blood until the water-soluble coating is dissolved by contact with blood. While the vasculature occlusive device disclosed in this patent includes an agent for enhancing thromboginicity of the device and also includes an outer coating to prevent such activity until the outer coating is dissolved by blood flow, there is no control over when the dissolving process begins and therefore no control over the time in which the thrombogenic agent becomes activated. Without such control, it is possible that thrombus can begin forming on the coil prior to the time the coil is properly placed within a vessel, or aneurysm, therefore making it very difficult if not impossible to reposition, or remove, the improperly placed coil. Additionally, with water-soluble outer protective coatings the passive process of removing the outer coating may be so slow that the reaction may not occur in a timely manner.

Still further, U.S. Patent No. 6,602,261, entitled, "Filamentous Embolic Device With Expansible Elements," discloses an embolic coil having embolizing elements placed along a filament, or coil, which are comprised of a hydrophilic, polymeric, hydrogel foam material, such as hydrogel foam. After implantation of this embolic coil within an aneurysm, the water-swellable foam begins to expand and more completely fill the aneurysm. While the expansible embolizing elements of this embolic coil, upon expansion, serve to more completely fill an aneurysm, there is again no control over when the expansible elements begin to expand. With no control over the time of expansion, the embolic coils may begin expanding prior to being properly placed within an aneurysm or may expand prior to the placement of multiple coils within an aneurysm thereby making it very difficult to properly place multiple coils within the aneurysm. After the expansion of the embolizing elements has occurred, it may be very difficulty, or even impossible to reposition the embolic coil.

Still further, U.S. Published Patent Application No. 2003/0093147, entitled, "Medical Devices That Stimulate Growth Factor Production," discloses a heart valve which is coated with a compound which stimulates the production of vascular endothelial growth factors (VEGF). This coating, which may take the form of hypoxia-inducible factor-1, stimulates bodily tissue to promote the proliferation of cells in the vicinity of the medical device and/or promotes the colonization of the medical device by endothelial cells to thereby decrease calcification around the medical device. Such VEGF production near the surface of the medical device reduces the risk of thrombosis and the long term need for anti-coagulation therapy. As with other prior art devices, there is no control over the time in which VEGF production starts and it is possible that such production may begin prior to the device being properly placed within the heart thereby making the proper placement of the device difficult.

### SUMMARY OF THE INVENTION

In accordance with one aspect of the present invention, there is provided a medical device, such as a vascular occlusive coil, which includes a support member which may take the form of a helical coil, a compound which stimulates tissue growth, such as vascular endothelial tissue growth, is disposed on the support member, and an outer barrier is disposed on the stimulation compound to prevent contact between the stimulation compound and bodily fluid when the medical device is inserted into a blood vessel or an aneurysm. The stimulation compound preferably takes the form of a vascular endothelial growth factor (VEGF), but may take the form of any biocompatible material which stimulates tissue growth. The outer barrier exhibits the characteristic of being inert to bodily fluid in that it is not water-soluble, but dissolves upon being exposed to an external agent. The external agent may take the form of a liquid medium which may be injected through a catheter at the site of the medical device.

In accordance with another aspect of the present invention, the stimulation compound may take the form of a basic fibroblast growth factor (bFGF) or a transforming growth factor β (TGFβ).

Alternatively, the stimulating compound may take the form of a material which stimulates the production of growth factors to in turn enhance tissue growth. One such material is a polypeptide which comprises hypoxia-inducible factor-1.

In accordance with another aspect of the present invention, the outer barrier preferably takes the form of an outer coating applied to the stimulation compound which prevents bodily fluid from reacting with the stimulation compound until such time as the outer barrier is exposed to the external agent. The external agent may take the form of a solvent which when applied to the outer coating through a catheter from a source outside of the body causes the outer coating to dissolve away from the stimulation compound.

In accordance with still another aspect of the present invention, there is provided a medical device, such as an vascular occlusive device, which includes an element comprised of a compound which stimulates tissue growth and an outer barrier applied to the stimulation element which prevents a reaction between bodily fluid and the stimulation element until such time as the outer barrier is dissolved away from the stimulation element to thereby expose the stimulation element to such bodily fluids.

There is discussed method for treating vascular disease which includes the steps of inserting a vascular occlusion device having a support member, a coating disposed on the support member which serves to stimulate tissue growth, and an outer barrier disposed on the stimulation coating which exhibits the characteristic of dissolving to uncover at least a portion of the stimulation coating when an external agent is applied to the outer barrier. The method includes the steps of inserting the vascular occlusive device into a blood vessel or an aneurysm and, upon election, applying an external agent through a catheter to the outer barrier to thereby cause the outer barrier to dissolve, or to otherwise be removed, and expose at least a portion, or all, of the stimulation coating to permit the stimulation coating to react with bodily fluids and stimulate tissue growth.

The method includes the steps of providing a medical device which has an element comprised of a compound which stimulates tissue growth, the stimulating element is coated with an outer barrier which exhibits the characteristic of dissolving to expose at least a portion of the stimulation compound when an external agent is applied to the outer barrier. This method step includes the steps of inserting the medical device into a blood vessel, and upon election, applying an external agent to the outer barrier to thereby cause the outer barrier to dissolve, or become removed and expose at least a portion of the stimulation element.

### BRIEF DESCRIPTION OF THE DRAWINGS

Figure 1 is an elevational view illustrating a medical device in the form of a vascular occlusive coil in accordance with one embodiment of the present invention;
Figure 2 is an elevational view, partly in cross-section illustrating the vascular occlusive coil as shown in Figure 1 illustrating a bioactive coating on the coil which stimulates vascular endothelial growth and an outer barrier coating in accordance with one embodiment of the present invention;
Figure 3 is an elevational view, partly in cross-section illustrating the vascular occlusive coil as shown in Figure 2 after the outer barrier coating has been removed and the stimulation coating is in a condition to react with bodily fluids, and,
Figures 4A through 4C illustrate the method steps of applying multiple vascular occlusive coils as shown in Figure 1 into an aneurysm and thereafter applying an external agent to thereby activate the stimulation coating.

### DESCRIPTION OF THE PREFERRED EMBODIMENT

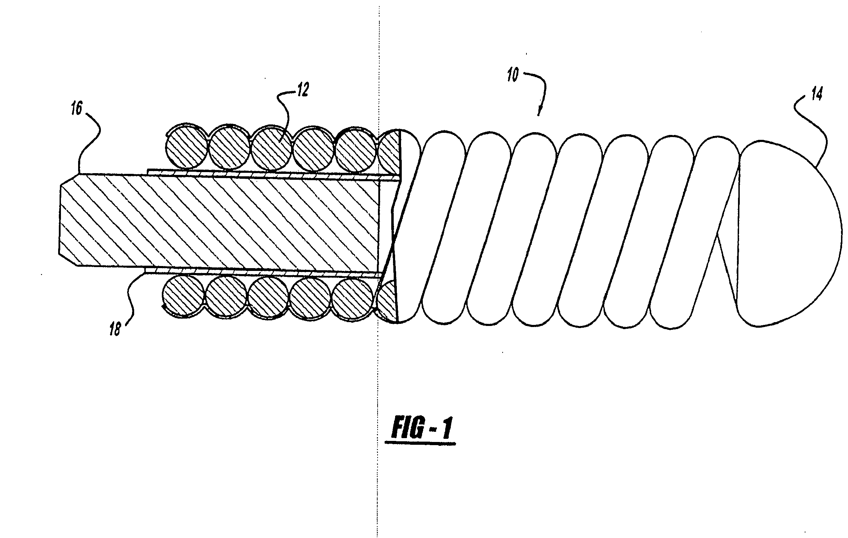

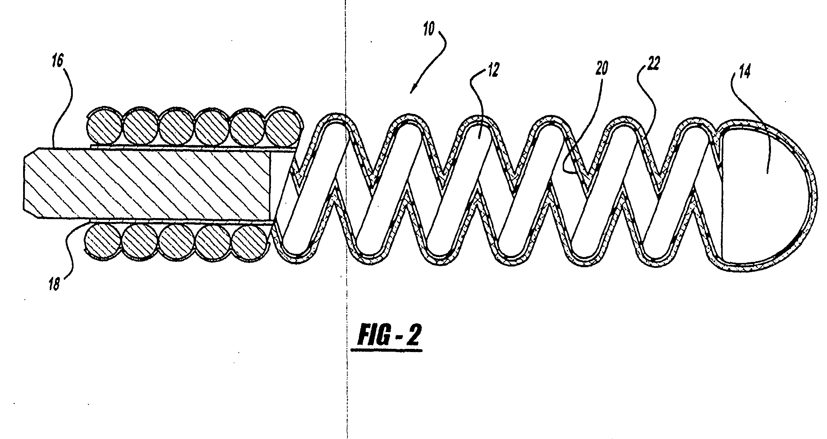

Figures 1 and 2 illustrate a preferred embodiment of a medical device such as an embolic coil 10 which may be placed along with other similar coils into a blood vessel or into an aneurysm in order to partially fill the aneurysm. More particularly, the embolic coil 10, or vascular occlusive coil, is a typical embolic coil which comprises a helically wound coil 12 formed from a platinum alloy wire wound into a helical configuration. The diameter of the wire is generally in the range of about 18 µm (0.0007 inches) to about 203 µm (0.008 inches) and the outer diameter of the coil 12 is preferably in a range of about 76 µm (0.003 inches) to about 1400 µm (0.055 inches). While the particular embolic coil 10 illustrated in Figures 1 and 2 is shown as being a straight, helically wound coil, it should be appreciated that embolic coils are formed in various configurations and may take the form of a helical wire wound in a helical configuration, a random shaped configuration or even a coil within a coil.

Preferably the embolic coil 10 includes a weld bead 14 which is attached to the distal end of the coil for providing a less traumatic distal end for the embolic coil 10. In addition, the embolic coil 10 includes a cylindrical headpiece 16 which is placed into the lumen of the helically wound coil 12 at the proximal end of the coil and is held in place by an adhesive material 18 interposed between the cylindrical headpiece 16 and the helically wound coil 12. The construction of the embolic coil 10 and an associated hydraulic deployment system for placing the embolic coil within an aneurysm is disclosed in more detail in U.S. Patent Application Serial No. 10/102,154, entitled, "Small Diameter Embolic Coil Hydraulic Deployment System," filed March 19, 2002, assigned to the same assignee of the present invention.

Figure 2 illustrates in more detail the embolic coil 10 which comprises the helically wound coil 12, a stimulation coating 20 comprised of a compound which stimulates tissue growth, such as a vascular endothelial growth factor (VEGF), disposed upon the coil 12. An outer barrier 22 is disposed upon the stimulation coating 20 for preventing the activation of the stimulation coating until such time as an election is made to activate the coating. More particularly, the stimulation coating 20, may take the form of any biocompatible material which when exposed to bodily fluids stimulates tissue growth. By "tissue growth" is meant cell proliferation, such as the proliferation of vascular endothelial cells, fibrosis formation or any other growth which enhances growth onto the embolic coil 12 and/or endothelialization of a blood vessel or an aneurysm.

While the stimulation coating 20 may take the form of a VEGF, there are numerous other compounds which would serve to stimulate tissue growth, such as a basic fibroblast growth factor (bFGF) or a transforming growth factor each of which would enhance fibrosis formation and endothelialization.

Alternatively, the stimulation coating 20 may take the form of a compound which itself would serve to induce the production of growth factors, such as vascular endothelial growth factors. One such compound which would serve to enhance the production of growth factors is hypoxia-inducible factor-1. Hypoxia-inducible factor-1 (HIF-1) is a heterodimer with two protein subunits, hypoxia-inducible factor 1-alpha (HIF-1α) and hypoxia inducible factor 1- beta (HIF-1β). HIF-1α stimulates production of vascular endothelial growth factor and is a protein factor that is found to be expressed in response to hypoxia. Hypoxia can elicit responses at systemic, local and cellular levels to increase oxygen delivery, or activate alternative metabolic pathways that do not require oxygen. Hypoxia response is described further in U.S. Patent No. 6,124, 131, entitled, "Mutant Hypoxia Inducible Factor-1 (HIF-1)".

The stimulation coating 20 serves to enhance tissue growth. Such tissue growth increases the effectiveness of the embolic coil 12 in occluding, or partially occluding, a vessel or an aneurysm. Preferably, the stimulation coating 20 is designed to be gradually released at a relatively slow rate so as to provide more effective stimulation of tissue growth.

The outer barrier 22 takes the form of a coating which is disposed on the bioactive stimulation coating 20 and serves to insulate the coating from adjacent bodily fluid until such time as a decision is made by a physician to activate the outer barrier 22 by applying an external agent to the barrier. The outer barrier 22 takes the form of a material which is inert to bodily fluid, but which dissolves and exposes the stimulation coating 20 when the outer barrier 22 is subjected to an external agent.

In a preferred embodiment, the outer barrier 22 is comprised of ethylene vinyl alcohol, and the external agent for dissolving the outer barrier 22 is comprised of dimethyl sulfoxide (DMSO). The external agent is preferably applied through a catheter from an external source to thereby dissolve the outer barrier 22 which in turn exposes the bioactive stimulation coating 20 to bodily fluids. It should be appreciated that there are numerous materials which could serve as a coating for stimulating vascular endothelial growth factor production which could serve, as an outer barrier and which could serve as an agent for dissolving or removing the outer barrier.

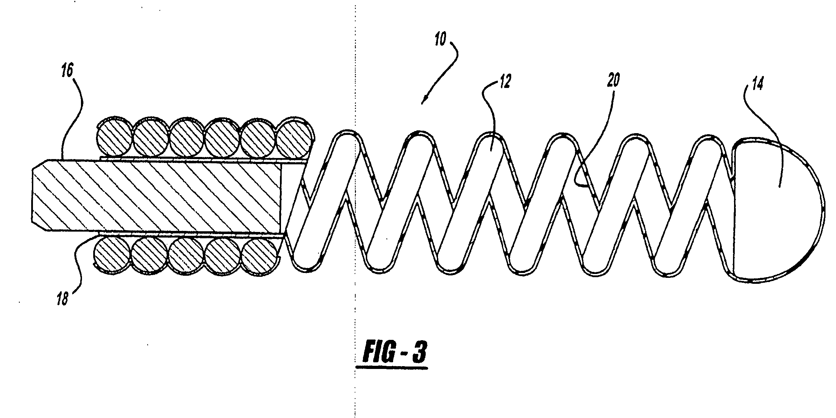

Figure 3 illustrates in more detail the embolic coil 10 after placement into an aneurysm and after the outer barrier 22 has been dissolved thereby exposing the stimulation coating 20 to bodily fluid, such as blood. The outer barrier is inert to bodily fluids, i.e., is not water-soluble, and the external agent, or solvent, is applied through a catheter from a source outside of the body to thereby dissolve, or remove, the outer barrier 22 for activation of the stimulation coating 20.

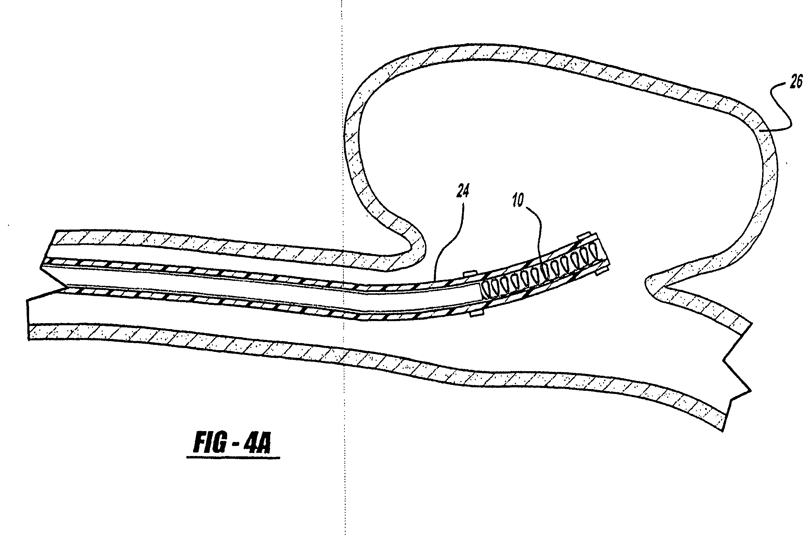

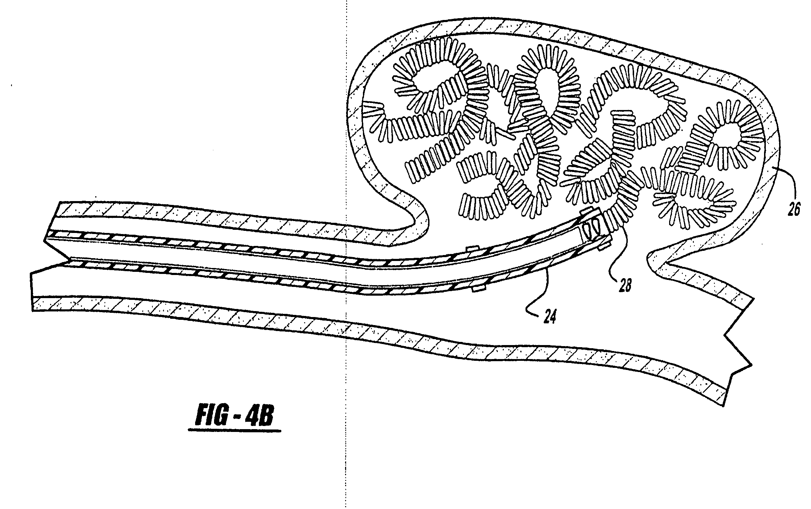

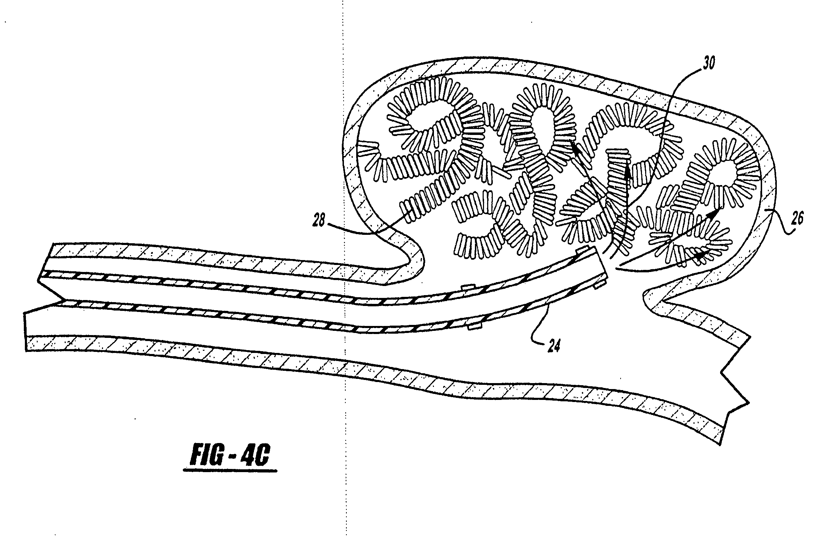

Figures 4A through 4C generally illustrate a method of utilizing the present invention. More particularly, Figure 4A illustrates a delivery catheter 24 having an embolic coil 10 placed in the distal end of the catheter for delivery into an aneurysm 26. Figure 4B illustrates the delivery catheter 24 being used to position multiple vascular occlusive coils including a final coil 28 into the aneurysm 26. Figure 4C illustrates the application of an external agent 30, which may take the form of a solvent, for dissolving the outer barrier 22, through a catheter from a source external of the body to thereby activate the stimulation coating 20.

It may be desirable to place all of the vascular occlusive coils into the aneurysm 26 prior to applying the external agent 30, however, another approach is that of placing a single coil into the aneurysm and thereafter activating that single coil, placing a second coil into the aneurysm and thereafter activating the second coil and so forth until all of the coils have been properly placed into the aneurysm. Once the coils are activated and tissue ingrowth commences it becomes more difficult to reposition the coil, therefore proper placement of the coils prior to activation becomes important. As may be appreciated, the advantage of the subject invention over prior devices is that the physician may determine at what point in time during the process of "filling" an aneurysm the physician elects to activate the coil or coils.

Although a preferred embodiment of the present invention has been described, it is to be understood that various modifications may be made by those skilled in the art without departing from the scope of the claims which follow.

## Claims

1. A vascular occlusion device (10) comprising:
a support member (12);
a compound (20) disposed on said support member (12) which stimulates tissue growth; and,
an outer barrier (22) disposed on said compound (20) to prevent exposure of said compound (20) to bodily fluids when said vascular occlusion device (10) is inserted into a blood vessel,
**characterised by** said outer barrier (22) exhibiting the characteristic of being inert to bodily fluid, in that it is not water-soluble, but dissolving when an external agent (30) is applied to said outer barrier (22).

2. A vascular occlusion device (10) as defined in Claim 1, wherein said compound (20) stimulates vascular endothelial tissue growth.

3. A vascular occlusion device (10) as defined in Claim 2, wherein said compound (20) comprises a vascular endothelial growth factor.

4. A vascular occlusion device (10) as defined in Claim 1, wherein said compound (20) comprises a basic fibroblast growth factor.

5. A vascular occlusion device (10) as defined in Claim 1, wherein said compound (20) comprises a transforming growth factor β.

6. A vascular occlusion device (10) as defined in Claim 2, wherein the outer barrier (22) takes the form of a coating applied to the support member (12).

7. A vascular occlusion device (10) as defined in Claim 6, wherein said outer barrier (22) is comprised of ethylene vinyl alcohol.

8. A vascular occlusion device (10) as defined in Claim 7, wherein said external agent (30) is comprised of dimethyl sulfoxide.

## Patentansprüche

1. Vaskuläre Okklusionsvorrichtung (10), die folgendes umfaßt:
ein Trägerelement (12);
eine auf dem Trägerelement (12) aufgebrachte Zusammensetzung (20), welche Gewebewachstum stimuliert; und
eine äußere Grenzschicht (22), die auf der Zusammensetzung (20) angeordnet ist, um zu verhindern, daß die Zusammensetzung (20) Körperfluiden ausgesetzt wird, wenn die vaskuläre Okklusionsvorrichtung (10) in ein Blutgefäß eingesetzt ist,
**dadurch gekennzeichnet, daß** die äußere Grenzschicht (22) die Eigenschaft hervorbringt, zu einem Körperfluid inert zu sein, **dadurch**, daß sie nicht wasserlöslich ist, sich jedoch löst, wenn ein externes Agens (30) auf die äußere Grenzschicht (22) angewandt wird.

2. Vaskuläre Okklusionsvorrichtung (10) nach Anspruch 1, wobei die Zusammensetzung (20) das Gewebewachstum des Gefäßendothels stimuliert.

3. Vaskuläre Okklusionsvorrichtung (10) nach Anspruch 2, wobei die Zusammensetzung (20) einen *Vascular Endothelial Growth Factor* umfaßt.

4. Vaskuläre Okklusionsvorrichtung (10) nach Anspruch 1, wobei die Zusammensetzung (20) einen *Basic Fibrolast Growth Factor* umfaßt.

5. Vaskuläre Okklusionsvorrichtung (10) nach Anspruch 1, wobei die Zusammensetzung (20) einen *Transforming Growth Factor* β umfaßt.

6. Vaskuläre Okklusionsvorrichtung (10) nach Anspruch 2, wobei die äußere Grenzschicht (22) die Form einer Beschichtung annimmt, die auf das Trägerelement (12) aufgebracht ist.

7. Vaskuläre Okklusionsvorrichtung (10) nach Anspruch 6, wobei die äußere Grenzschicht (22) einen Ethylenvinylalkohol umfaßt.

8. Vaskuläre Okklusionsvorrichtung (10) nach Anspruch 7, wobei das externe Agens (30) Dimethylsulfoxid umfaßt.

## Revendications

1. Dispositif d'occlusion vasculaire (10) comprenant :
■ un élément de support (12) ;
■ un composé (20) disposé sur ledit élément de support (12) qui stimule la croissance tissulaire ; et
■ une barrière externe (22) disposée sur ledit composé (20) pour empêcher l'exposition dudit composé (20) sur les fluides corporels lorsque ledit dispositif d'occlusion vasculaire (10) est inséré dans un vaisseau sanguin,
**caractérisé par** ladite barrière externe (22) laissant apparaître la caractéristique d'être inerte aux fluides corporels, en ce qu'elle n'est pas soluble dans l'eau, mais se dissolvant lorsqu'un agent externe (30) est appliqué sur ladite barrière externe (22).

2. Dispositif d'occlusion vasculaire (10) selon la revendication 1, dans lequel ledit composé (20) stimule la croissance tissulaire endothéliale vasculaire.

3. Dispositif d'occlusion vasculaire (10) selon la revendication 2, dans lequel ledit composé (20) comprend un facteur de croissance endothéliale vasculaire.

4. Dispositif d'occlusion vasculaire (10) selon la revendication 1, dans lequel ledit composé (20) comprend un facteur de croissance de fibroblaste basique.

5. Dispositif d'occlusion vasculaire (10) selon la revendication 1, dans lequel ledit composé (20) comprend un facteur de croissance transformant β.

6. Dispositif d'occlusion vasculaire (10) selon la revendication 2, dans lequel la barrière externe (22) prend la forme d'un revêtement appliqué sur l'élément de support (12).

7. Dispositif d'occlusion vasculaire (10) selon la revendication 6, dans lequel ladite barrière externe (22) est composée d'alcool éthylène vinyle.

8. Dispositif d'occlusion vasculaire (10) selon la revendication 7, dans lequel ledit agent externe (30) est composé de diméthyl sulfoxide.
